(19) Europäisches Patentamt
European Patent Office
Office européen des brevets

(11) **EP 2 452 190 B1**

(12) **EUROPÄISCHE PATENTSCHRIFT**

(45) Veröffentlichungstag und Bekanntmachung des Hinweises auf die Patenterteilung:
**16.11.2016 Patentblatt 2016/46**

(21) Anmeldenummer: **10731466.8**

(22) Anmeldetag: **07.07.2010**

(51) Int Cl.:
*G01N 33/50* *(2006.01)* *G01N 33/52* *(2006.01)*
*B01L 3/00* *(2006.01)* *G01N 27/00* *(2006.01)*

(86) Internationale Anmeldenummer:
**PCT/EP2010/004090**

(87) Internationale Veröffentlichungsnummer:
**WO 2011/003583 (13.01.2011 Gazette 2011/02)**

(54) **SENSOR ALS BIOCHIP**

SENSOR AS BIOCHIP

DÉTECTEUR SOUS FORME DE BIOPUCE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **07.07.2009 EP 09008858**

(43) Veröffentlichungstag der Anmeldung:
**16.05.2012 Patentblatt 2012/20**

(73) Patentinhaber: **Siemens Aktiengesellschaft 80333 München (DE)**

(72) Erfinder:
• **VOEROES, Janos**
  **CH-8050 Zürich (CH)**
• **SUGIHARA, Kaori**
  **CH-8045 Zürich (CH)**
• **ZAMBELLI, Tomaso**
  **CH-8006 Zürich (CH)**

• **NIRSCHL, Martin**
  **81739 München (DE)**

(56) Entgegenhaltungen:
**WO-A2-2006/076008 DE-A1- 10 308 975**

• **TIEN H T ET AL: "Supported planar lipid bilayers (s-BLMs) as electrochemical biosensors" ELECTROCHIMICA ACTA, ELSEVIER SCIENCE PUBLISHERS, BARKING, GB LNKD-DOI:10.1016/S0013-4686(98)00107-8, Bd. 43, Nr. 23, 30. Juli 1998 (1998-07-30) , Seiten 3587-3610, XP004133579 ISSN: 0013-4686**
• **Batsios G.: "Optical membrane permeability sensor" Juli 2009 (2009-07), Seiten 1-32, XP002600546 Gefunden im Internet: URL:http://www.lbb.ethz.ch/Publications/Diploma_semester_works/GBatsios_SemesterProj ect [gefunden am 2010-09-13]**

**Beschreibung**

Die vorliegende Erfindung betrifft einen Sensor, insbesondere einen Biochip zur Anwendung in miniaturisierten Laboren, wie beispielsweise auf so genannten lab-on-a-chip-Karten.

Biochips sind Vorrichtungen durch die kleinste Mengen biologischen Materials detektiert und/oder untersucht werden können. Durch Biochips können beispielsweise Moleküle wie Nukleinsäuren oder Proteine auf festen Oberflächen adsorbiert werden und machen eine automatisierte, schnelle und parallele Analyse der Proben möglich.

Grundsätzlich umfasst ein Biochip deshalb ein Testfeld, einen Sensor und eine Auswerteelektronik, wobei letztere auch extern angeordnet und durch Anschlüsse mit dem Sensor auf dem Chip gekoppelt sein kann.

Das Testfeld ist normalerweise durch eine Art Nano-Reservoir gekennzeichnet, das zur Sorption der Probe dient.

Ein Beispiel eines Sensors für einen Biochip ist aus der DE 103 08 975 B4 bekannt, dort ist ein Dünnfilmresonator, bei dem die Elektrodenschicht, die piezoelektrische Schicht und die weitere Elektrodenschicht schichtförmig übereinander gestapelt sind, offenbart. Die piezoelektrische Schicht besteht beispielsweise aus Zinkoxid. Die obere Elektrodenschicht (top electrode) ist aus Gold und weist die Anlagerungsfläche zur Anlagerung (z.B. Adsorption) der Substanz eines Fluids auf. Über die untere Elektrodenschicht (bottom electrode) ist der Dünnfilmresonator auf einem Siliziumsubstrat aufgebracht. Zur akustischen Entkopplung des Siliziumsubstrats und des Dünnfilmresonators voneinander ist dazwischen beispielsweise ein akustischer Spiegel aus $\lambda/4$-dicken Schichten unterschiedlicher akustischer Impedanz angeordnet.

Mit den bekannten Biochips ist es möglich, Substanzen mit relativ hoher Masse, wie beispielsweise Makromoleküle wie Proteine oder Nukleinsäuren, auf den momentan üblichen Testfeldern zu adsorbieren. Durch ihre Masse bewirken diese Moleküle ein verändertes Schwingungsverhalten des Testfeldes und können entsprechend detektiert werden. Bei kleinen Molekülen mit geringer Masse funktioniert dieses System nur schlecht (also mit geringer Auflösung) oder überhaupt nicht.

Für die Detektion kleinerer Moleküle oder Atome oder Ionen sind viele Techniken bekannt, aber keine die auf dem System eines piezoakustischen Dünnfilmresonators, wie er aus der DE 103 08 975 beispielsweise bekannt ist, beruht.

Das Dokument "Supported planar lipid bilayers (s-BLMs) as electrochemical biosensors" (H.T. Tien and A.L. Ottova; ELECTROCHEMICA ACTA, Vol. 43, No. 23, pp. 3587-3610, 1998, ELSEVIER SCIENCE PUBLISHERS) offenbart einen Biosensor mit einer von Metall und Hydrogel unterstützten Lipid-Doppelschicht-Membran.

Die WO 2006/076008 A2 zeigt eine Auswahl an Neuralstimulationsanordnungen. Die Anordnungen umfassen eine aufnehmende Komponente, welche Mittel beinhaltet, um selektiv eine stimulierende Spezies in die Anordnung zu transportieren. Außerdem umfassen die Anordnungen eine freisetzende Komponente, welche Mittel beinhaltet, um die Spezies freizusetzen, und Mittel, um eine Konzentrationsgradienten einer zweiten Spezies zu produzieren.

Aufgabe der vorliegenden Erfindung ist es, Sensoren derart zu verbessern, dass die Massensensitivität gesteigert wird.

Die Lösung der Aufgabe wird in der vorliegenden Beschreibung, den Ansprüchen und den Figuren offenbart.

Demnach ist Gegenstand der Erfindung ein Biochip mit einem Sensor und einem Testfeld, wobei auf das Testfeld ein Reservoir, also eine absorbierende Schicht und darüber eine Membran aufgebracht ist, derart, dass die Membran eine Doppellipidschicht, eine Zelle, eine Zellwand und/oder eine Zellmembran umfasst, wobei durch die Kombination des Sensors mit dem Reservoir und der Membran eine Durchlässigkeit von Substanzen durch Membranen untersuchbar ist.

Es wurde festgestellt, dass Zellmembranen, Zellwände, Zellen und /oder Doppellipidschichten eine große Vielfalt an Membranproteinen haben, die die Zelladhäsion bewirken. Diese Membranen haben für verschiedene Substanzen unterschiedliche Durchlässigkeiten, so dass durch die Membran, insbesondere wenn sie noch mit bestimmten Membranproteinen ausgestattet ist, nur bestimmte Substanzen durchwandern können. Dieser Effekt wird erfindungsgemäß zur Detektion von Substanzen ausgenutzt.

In den Zellmembranen, Zellwänden, Doppellipidschichten und/oder Zellen gibt es verschiedene Pfade für Moleküle, Ionen, und Atome. Darunter befinden sich beispielsweise auch so genannte Ionenkanäle, die in den Membranen Poren bilden, die kleine Moleküle, wie z.B. Ionen aufnehmen.

Es ist gelungen, Ionenkanäle in künstlich synthetisierten Doppellipidschichten aufzubauen. Damit wird die Zellmembrandurchlässigkeit nachempfunden. Gemäß der Erfindung werden diese Doppellipidschichten beispielsweise als planare Plasmamembranen beispielsweise auf polymere Träger aufgebracht, die die Membranen zum einen stabilisieren und zum anderen die zu detektierende Substanz absorbieren.

Nach einer vorteilhaften Ausführungsform ist die Durchlässigkeit der Membran an die zu detektierende Substanz angepasst. Beispielsweise handelt es sich dabei um eine ionenselektive Membran.

Die Membranen und die polymeren Träger werden so gewählt, dass, wenn die gesuchte Substanz in die Membranen und den polymeren Träger eindringt und dort absorbiert wird, sie (nur der polymere Träger, nicht zwingend die Membran) ihre physikalischen Eigenschaften ändern, so dass die Substanz detektiert werden kann. Beispielsweise kann sich der Brechungsindex, die magnetische Induktion, die Viskosität -oder die akustische Durchlässigkeit des Aufbaus durch das Vorhandensein der Testsubstanz ändern. Diese Änderung kann dann durch den Sensor detektiert werden.

Nach einer vorteilhaften Ausführungsform ist vorgesehen, dass innerhalb der Membran noch Proteine so genannte Membranproteine zur Anlagerung der gesuchten Moleküle oder Ionen angeordnet sind.

Nach einer vorteilhaften Ausführungsform der Erfindung ist vorgesehen, dass das Reservoir Poren bildet. Dabei ist vorteilhaft, wenn das poröse Material ein Polymer ist, insbesondere eine Polelectrolyte-Multilayerpolymer, ein Gel oder eine andere poröse Masse wie poröse Metalle oder Keramiken, beispielsweise poröses Silizium, Aluminium oder ähnliches.

Im Falle eines PEM polymeren Trägers ist es bevorzugt, wenn die PEM durch eine Schicht-für-Schicht-Methode mit verschiedenen Polyelektrolyten aufgebaut wird. Beispielsweise können hier Polyelektrolyten wie Polyetherimid (PEI), Polyallylamin (PAH), PGA (Polyglutamatsäure) oder PSS (Polystyrolsulfonat).

Nach einer vorteilhaften Ausführungsform der Erfindung ist der Sensor ein optischer, akustischer, magnetischer oder mechanischer Sensor, wie beispielsweise Feldeffekttransistoren, OWL (Optical waveguide lightmode) - Spektroskopie, QCM (Quartz crystal microbalance) Kristalle oder GMR (Giant Magnetoresistance) Sensoren.

Nach einer weiteren vorteilhaften Ausführungsform der Erfindung werden die Membranen durch DOPS, DOPA DOPC direkt aus Zellen oder Lipide gewonnen. Diese können dann direkt auf das poröse Material des Reservoirs aufgebracht werden.

Nach einer vorteilhaften Ausführungsform sind in der Membran noch Proteine eingearbeitet, wobei diese Proteine durch Proteoliposome, direkt, als Lösung oder mechanisch eingebracht werden können.

Der Sensor gemäß der Erfindung kann ein optischer, ein akustischer, ein magnetischer oder ein sonstiger Sensor sein.

Nach einer bevorzugten Ausführungsform ist der Sensor ein akustischer Resonator wie der aus der DE 103 08 975 B4 bekannte. Dazu wird auf den dort beschriebenen Aufbau, insbesondere auf den Oberflächenabschnitt (8) ein Reservoir, insbesondere ein poröses Reservoir angeordnet, auf das dann die Membran aufgebracht wird. Insbesondere kann dadurch eine Veränderung der Viskosität, oder eine damit einhergehenden Veränderung der Eindringtiefe von akustischen Wellen detektiert werden.

Ebenso gut kann das Reservoir mit der Membran auf Testfelder für QCM-D oder OWLS-Techniken aufgebracht werden. Insbesondere kann dadurch das Quellen oder Zusammenziehen der PEM detektiert werden.

Im Folgenden wird die Erfindung noch anhand zweier Figuren, die eine beispielhafte Ausführungsform der Erfindung zeigen, näher erläutert:

Figur 1    zeigt den Aufbau eines akustischen Resonators gemäß der Erfindung schematisch;

Figur 2    zeigt die gleiche Ausführungsform wobei das Testfeld überdimensional groß gezeigt wird;

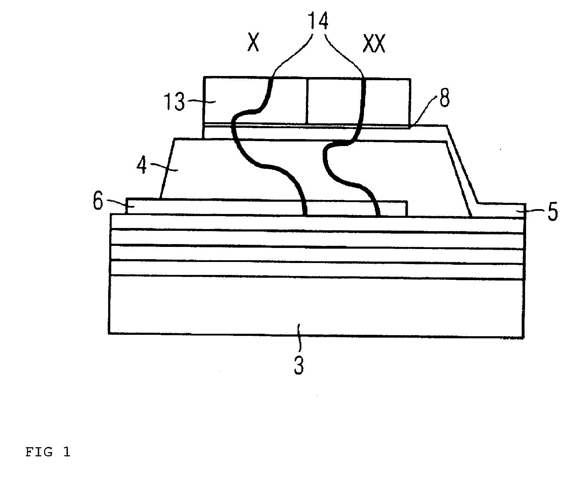

In Figur 1 ist der Aufbau gemäß der DE 103 08 975 zu sehen, insbesondere das Substrat 3 mit beispielsweise einem akustischen Spiegel zur Verstärkung des Signals, darauf die erste Elektrode 6, die piezoelektrische Schicht 4, die obere Elektrode 5 und der Oberflächenabschnitt 8. Auf dem Oberflächenabschnitt oder Testfeld 8 ist gemäß der Erfindung eine zusätzliche Schicht 13 vorgesehen, die links (X) ungefüllt (d.h. in Abwesenheit der zu detektierenden Substanz) und rechts (XX) mit der zu detektierenden Substanz gefüllt zu sehen ist.

Schematisch gezeigt wird hier der Verlauf der akustischen Welle 14, die bei X, dem ungefüllten Reservoir in die Schicht 13 deutlich eindringt, wohingegen die akustische Welle im gefüllten Reservoir XX nahezu verschwunden ist. Das zeigt, dass im gefüllten Reservoir XX die akustische Welle nicht so weit eindringt wie im ungefüllten Reservoir X.

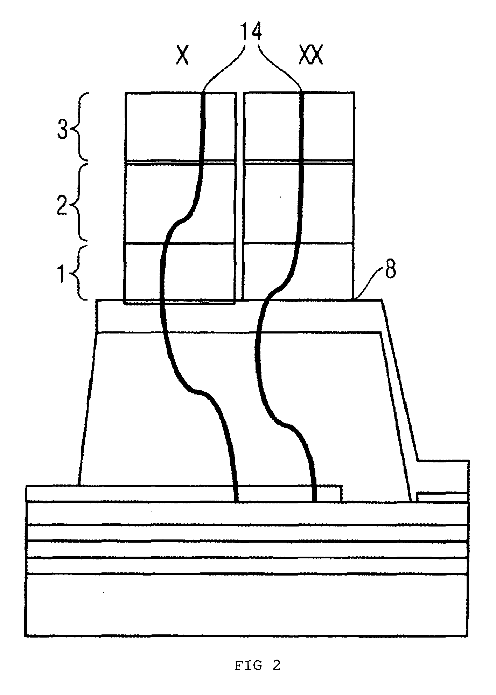

In Figur 2 ist dieser Effekt noch genauer zu sehen. Wieder erkennt man den aus der Figur 1 bekannten Aufbau, allerdings wird hier der Verlauf der akustischen Welle noch deutlicher gezeigt. Die Welle kommt durch die obere Elektrode im Testfeld 8 an, wobei sie im Fall X, dem ungefüllten Reservoir, noch bis zur Mitte der Schicht als Welle erhalten bleibt, wohingegen sie beim Reservoir XX, das mit der zu detektierenden Substanz gefüllt ist, nicht weiter als ins untere Drittel der Schicht eindringt.

Den Unterschied in der akustischen Durchlässigkeit des gefüllten und ungefüllten Materials kann der akustische Resonator detektieren. Dies geschieht dadurch, dass in einem Fall (X) die Masse der Schicht des mittleren Drittels vom Sensor detektiert wird und damit zur Änderung der Resonanzfrequenz beiträgt und im anderen Fall (XX) die Masse des mittleren Drittels der Schicht nicht von der akustischen Welle durchdrungen wird und dadurch nicht zu einer Verschiebung der Resonanzfrequenz beiträgt.

Beispielsweise ändert die zu detektierende Substanz die Durchlässigkeit $\delta$ der akustischen Welle.

Diese Änderung der akustischen Durchlässigkeit folgt der durch die nachstehende Formel wiedergegebenen Gesetzmäßigkeit:

$$\delta = \sqrt{2\eta / \rho\omega}$$

wobei

$\eta$ = Viskosität
$\rho$ = Dichte

ω = die Winkelfrequenz

bedeutet.

So ändert die sorbierte Substanz die Viskosität und damit die Durchlässigkeit für akustische Wellen und bewirkt damit eine Frequenzänderung, da die Masse des Reservoirs die im gefüllten Reservoir nicht durchlaufen wird, direkt proportional zu der Signaländerung ist.

Die Dicke der Schicht 13, also des Reservoirs inklusive der Membran, ist vorzugsweise größer als die Eindringtiefe der akustische Welle, damit zusätzliche Massen wie unspezifisch adsorbierte Teilchen wie Schmutz nicht zum Messsignal beitragen.

Die Substanzen, die untersucht werden sollen, können je nach Aggregatszustand flüssig, gasförmig oder auch als Lösung fester Verbindungen detektiert werden.

Durch die vorliegende Erfindung wird es mit besonders hoher Sensitivität möglich, kleine und kleinste Moleküle, wie auch Ionen oder Atome, zu detektieren. Dies geschieht einfach mittels des bekannten akustischen Resonators FBAR oder mittels anderer die physikalischen Eigenschaften der gefüllten Schicht messenden Techniken. Durch die Kombination eines Sensors mit dem Reservoir und der Membran kann die Durchlässigkeit von Substanzen (z.B. Wirkstoffen) durch Membranen wie Zellmembranen, Doppellipidschichten und Zellwände untersucht werden.

## Patentansprüche

1. Biochip mit einem Sensor und einem Testfeld (8), wobei auf das Testfeld ein Reservoir, also eine absorbierende Schicht(13) angeordnet ist, auf das eine Membran aufgebracht ist, derart, dass die Membran eine Doppellipidschicht, eine Zelle, eine Zellwand und/oder eine Zellmembran umfasst, wobei durch die Kombination des Sensors mit dem Reservoir und der Membran eine Durchlässigkeit von Substanzen durch Membranen untersuchbar ist.

2. Biochip nach Anspruch 1, bei dem das Reservoir ein poröses Material umfasst.

3. Biochip nach einem der vorstehenden Ansprüche, wobei die Durchlässigkeit der Membran an die detektierende Substanz angepasst ist.

4. Biochip nach einem der vorstehenden Ansprüche, wobei das Reservoir aus einem Material, ausgewählt aus der Gruppe der Polymere, Gele und porösen keramischen oder metallischen Materialien ausgewählt ist.

5. Biochip nach einem der vorstehenden Ansprüche, wobei der Sensor ausgewählt ist aus der Gruppe der akustischen, optischen, magnetischen und/oder mechanischen Sensoren.

6. Biochip nach einem der vorstehenden Ansprüche, wobei das Reservoir aus einem mehrschichtigen Polymer ist.

7. Biochip nach einem der vorstehenden Ansprüche, wobei die Membran auf dem Reservoir direkt aus Zellen oder Doppellipidschichten aufgebaut ist.

8. Biochip nach einem der vorstehenden Ansprüche, wobei innerhalb der Membran Proteine vorgesehen sind, die die Durchlässigkeit der Membran beeinflussen.

9. Biochip nach einem der vorstehenden Ansprüche, wobei der Sensor ein akustischer Resonanzsensor mit einer piezoelektrischen Schicht (4) ist.

10. Biochip nach Anspruch 9, wobei die Dicke des Reservoirs und der Membran größer als die Eindringtiefe der akustischen Welle ist.

11. Biochip nach einem der Ansprüche 1-8, wobei der Sensor auf eine Änderung der optischen Eigenschaften des Testfelds reagiert.

## Claims

1. Biochip comprising a sensor and a test panel (8), wherein a reservoir, i.e. an absorbent layer (13), is arranged on the test panel, onto which reservoir a membrane is applied, such that said membrane comprises a lipid bilayer, a cell, a cell wall and/or a cell membrane, wherein the combination of the sensor with the reservoir and the membrane means that a permeability of substances by membranes can be examined.

2. Biochip according to claim 1, wherein the reservoir comprises a porous material.

3. Biochip according to one of the preceding claims, wherein the permeability of the membrane is adapted to the substance that must be detected.

4. Biochip according to one of the preceding claims, wherein the reservoir is made of a material that is selected from the group comprising polymers, gels and porous ceramic or metallic materials.

5. Biochip according to one of the preceding claims, wherein the sensor is selected from the group comprising acoustic, optical, magnetic and/or mechanical sensors.

6. Biochip according to one of the preceding claims, wherein the reservoir comprises a multilayer polymer.

7. Biochip according to one of the preceding claims, wherein the membrane on the reservoir is composed directly of cells or lipid bilayers.

8. Biochip according to one of the preceding claims, wherein proteins that influence the permeability of the membrane are provided within the membrane.

9. Biochip according to one of the preceding claims, wherein the sensor is an acoustic resonance sensor comprising a piezoelectric layer (4).

10. Biochip according to claim 9, wherein the thickness of the reservoir and the membrane is greater than the penetration depth of the acoustic wave.

11. Biochip according to one of claims 1 to 8, wherein the sensor reacts to a change in the optical properties of the test panel.


**Revendications**

1. Biopuce avec un capteur et un champ de test (8), dans laquelle, sur le champ de test, sont disposés un réservoir, également une couche absorbante (13), sur lequel réservoir est appliquée une membrane, de telle sorte que la membrane comprenne une bicouche lipidique, une cellule, une paroi cellulaire et/ou une membrane cellulaire, dans laquelle une perméabilité de substances à travers des membranes peut être analysée par la combinaison du capteur avec le réservoir et la membrane.

2. Biopuce selon la revendication 1, dans laquelle le réservoir comprend un matériau poreux.

3. Biopuce selon l'une des revendications précédentes, dans laquelle la perméabilité de la membrane est adaptée à la substance de détection.

4. Biopuce selon l'une des revendications précédentes, dans laquelle le réservoir est sélectionné parmi un matériau sélectionné dans le groupe des polymères, des gels et des matériaux métalliques ou céramiques poreux.

5. Biopuce selon l'une des revendications précédentes, dans laquelle le capteur est sélectionné dans le groupe des capteurs acoustiques, optiques, magnétiques et/ou mécaniques.

6. Biopuce selon l'une des revendications précédentes, dans laquelle le réservoir est en polymère multicouche.

7. Biopuce selon l'une des revendications précédentes, dans laquelle la membrane est constituée sur le réservoir directement à partir de cellules ou de bicouches lipidiques.

8. Biopuce selon l'une des revendications précédentes, dans laquelle, à l'intérieur de la membrane, sont prévues des protéines qui influencent la perméabilité de la membrane.

9. Biopuce selon l'une des revendications précédentes, dans laquelle le capteur est un capteur à résonance acoustique avec une couche piézoélectrique (4).

10. Biopuce selon la revendication 9, dans laquelle l'épaisseur du réservoir et de la membrane est supérieure à la profondeur de pénétration de l'onde acoustique.

11. Biopuce selon l'une des revendications 1 à 8, dans laquelle le capteur réagit à une modification des propriétés optiques du champ de test.

FIG 1

FIG 2

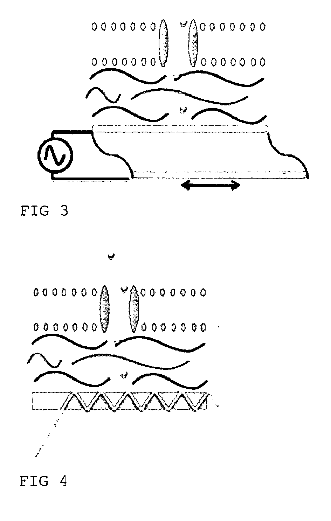

FIG 3

FIG 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10308975 B4
- DE 10308975

- WO 2006076008 A2

**In der Beschreibung aufgeführte Nicht-Patentliteratur**

- Supported planar lipid bilayers (s-BLMs) as electro-chemical biosensors. **H.T. TIEN ; A.L. OTTOVA.** ELECTROCHEMICA ACTA. ELSEVIER SCIENCE PUBLISHERS, 1998, vol. 43, 3587-3610